# EUROPEAN PATENT SPECIFICATION

(11) **EP 3 445 294 B1**
(45) Date of publication and mention of the grant of the patent: **04.12.2024**
(21) Application number: 17720054.0
(22) Date of filing: 20.04.2017
(51) Int. Cl.: A61F 2/90, A61F 2/915, A61B 5/00

(54) **TISSUE STENT**
GEWEBESTENT
ENDOPROTHÈSE TISSULAIRE

(30) Priority: 20.04.2016 LU 93035
(43) Date of publication of application: 27.02.2019
(73) Proprietor: Technische Universität Hamburg-Harburg, 21073 Hamburg (DE); Universitätsklinikum Hamburg-Eppendorf (UKE), 20246 Hamburg (DE); First Sensor Microelectronic Packaging GmbH, 01109 Dresden (DE)
(72) Inventor: ADAM, Gerhard, 22393 Hamburg (DE); BRAUNSCHWEIG, Markus, 01109 Dresden (DE); BUHK, Jan-Hendrik, 20535 Hamburg (DE); BIBIN, John, 21073 Hamburg (DE); KOOPS, Andreas, 14050 Berlin (DE); KRAUTSCHNEIDER, Wolfgang, 21149 Hamburg (DE); RANJAN, Rajeev, 21073 Hamburg (DE); SPINK, Clemens, 22529 Hamburg (DE); SCHRÖDER, Dietmar, 29525 Uelzen (DE); WOLDT, Gregor, 01187 Dresden (DE)
(74) Representative: RCD-Patent Giesen, Schmelcher & Griebel Patentwanwälte PartG mbB
(86) International application number: PCT/EP2017/059442
(87) International publication number: WO 2017/182588

(56) References cited:
- WO-A1-2013/164829
- WO-A1-2016/050972
- WO-A2-2008/011592
- FR-A1- 2 905 260
- US-A1- 2002 183 628

## Description

### Background

Within medicine it is known that hypertensions in lumen as well as in tissue may lead to severe problems.

It is known form WO 2013/164829 to provide a stent with electrodes to provide electrical stimulation of a blood vessel in order to counteract certain medical conditions.

For example it is known that hypertension in the vena portae hepatis (engl. Hepatic portal vein) or in any branch thereof may be caused by changes of the liver tissue leading to obstructions e.g. as a result of cirrhosis of the liver, or a liver carcinoma (primary cancer or secondary cancer) or a (chronic) hepatitis just to mention a few.

To compensate for the portal hypertension natural shunts are developing shunting arterial blood via veins towards the heart.

Other comparable situations are known as varix / varicose veins.

It is known that the shunt / varicose veins may also be found at other locations such as the gastrointestinal tract or the esophagus.

These veins are prone to leakage which may be live threatening as most of these veins are located within the body where treatment and / or discovery of a leakage is quite delicate.

In order to compensate portal hypertension, it has been proposed to introduce transjugular intrahepatic portosystemic shunts, i.e. lumen shunts. These artificial shunts reduce the hypertension and thereby counteract the development of "natural" shunts. These shunts may be provided by minimal invasive surgery.

However, it is known that theses shunts are prone to increasing occlusion. Such occlusions are reported to occur within a short time period, such that after a 12 month period up to 50 % of these stent grafts are occluded by stenosis or other thrombotic material.

Such condition again affords intervention. Since any intervention is traumatic and risky, such anew interventions are delayed as much as possible but needs regular control of the passage through the stent graft.

Such regular control involves doppler- sonography which necessitates a lot of practice to provide reliable results. As interpretation of these sonograms is rather complex, the results are hardly comparable from one medical practitioner to another. Also such sonographic examinations are quite expensive.

Hence, it would be preferable if a method and devices would exist allowing for precise measurements at low costs.

### Short description of the invention

To overcome one or more problems in the art, the invention proposes a tissue stent for allowing passage of a fluid, the tissue stent being suited for implantation into the body of a mammal.

The tissue stent according to the invention (the present invention is as set forth in the appended claims) comprises a body forming said passage from an entry side towards an exit side when implanted. The tissue stent according to the invention comprises also at least two sensing portions, whereby a first sensing portion is arranged near the entry side and whereby a second sensing portion is arranged near the exit side, the first sensing portion and second sensing portion being pressure sensors. The tissue stent according to the invention comprises an energy harvesting and storage portion and a wireless sending portion for sending information indicative of the functional conditions of the passage, whereby the at least two sensing portions and the wireless sending portion are energized via the energy harvesting and storage section.

Further details are subject to the dependent claims and the description.

Short summary of the figures.

In the following detailed description reference will be made towards the figures, in which
Fig. 1 shows a schematic overview of a tissue stent according to the invention,
Fig. 2 shows another schematic overview of the tissues stent according to figure 1,
Fig. 3 shows another schematic overview of a tissue stent according to another aspect of the invention,
Fig. 4 shows a schematic cross-sectional view of a tissue stent according to still another aspect of the invention,
Fig. 5 shows a further aspect of embodiments of the invention,
Fig. 6 shows another aspect of embodiments of the invention,
Fig. 7 shows yet another aspect of embodiments of the invention,
Fig. 8 shows yet another aspect of embodiments of the invention,
Fig. 9 shows yet another aspect of embodiments of the invention as set forth in the appended claims,
Fig. 10 shows another aspect of the invention,
Fig. 11 shows an embodiment of the aspect of Figure 10 in greater detail, and
Fig. 12 shows another embodiment of the aspect of Figure 10 in greater detail.

### Detailed description

The present disclosure describes preferred embodiments with reference to the Figures, in which like reference signs represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, method steps or characteristics of the invention may be combined in any suitable manner in one or more embodiments. In the description, numerous specific details are recited to provide a thorough understanding of embodiments of the invention.

I.e., unless indicated as alternative only any feature of an embodiment may also be utilized in another embodiment.

In addition, even though at some occurrences certain features will be described with reference to a single entity, such a description is for illustrative purpose only and actual implantations of the invention may also comprise one or more of these entities. I.e. usage of singular also encompasses plural entities unless indicated.

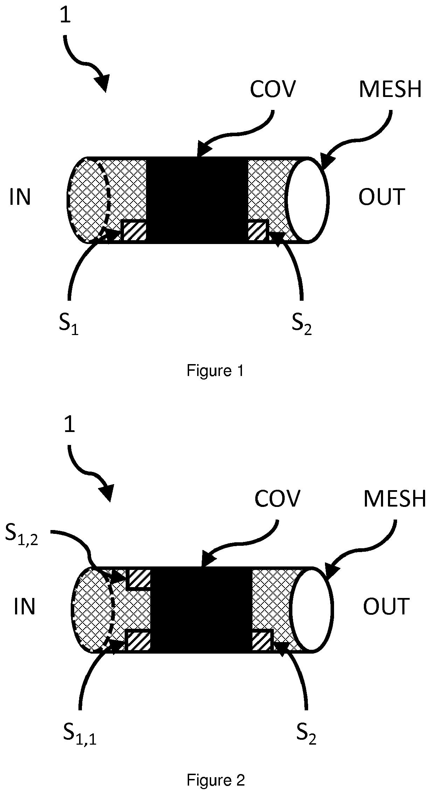

A tissue stent according to the invention is shown schematically in Figure 1.

A tissue stent 1 according to the invention allows for passage of a fluid such as blood, the tissue stent 1 is therefore suited for implantation into the body of a mammal, e.g. composed of biocompatible materials or covered by biocompatible material.

Although described with reference to blood the concept offered by the invention may be used with respect to other fluids as well. In addition, although described with respect to determining a passage, it is clear to a person skilled in the art that the principles of the invention may as well be used for determining a partial or full blockage of the passage.

The tissue stent 1 according to the invention comprises a body forming said passage from an entry side IN towards an exit side OUT when implanted.

The tissue stent 1 according to the invention comprises also at least two sensing portions S₁, S₂, whereby a first sensing portion S₁ is arranged near the entry side IN and whereby a second sensing portion S₂ is arranged near the exit side OUT, the first sensing portion S₁ and second sensing portion S₂ being pressure sensors Sₚᵣₑₛₛᵤᵣₑ.

It is to be understood that IN and OUT are merely of descriptive nature as blockage may be determined as a pressure gradient / pressure difference relative to the first sensing portion S₁ and the second sensing portion S₂.

Furthermore, it is also to be understood that a first sensing portion S₁ and a second sensing portion S₂ is not be held as limiting but any number of sensing portions greater and equal to two may be used. E.g. as shown in Figure 2, when more than one sensor portion is used, e.g. S_{1,1} and S_{1,2}, at like positions, these sensor portions S_{1,1} and S_{1,2} may allow for more reliability in that a cloth on one sensor portion may not jeopardize the results.

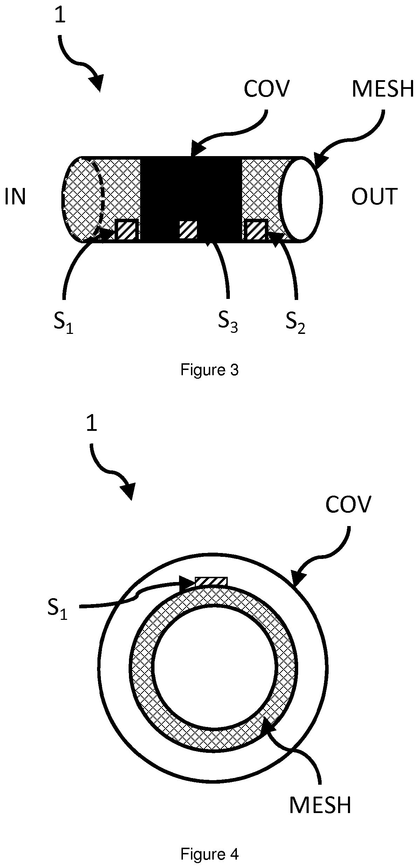

On the other hand if a third sensor S₃ is place in between the first sensing portion S₁ and the second sensing portion S₂, e.g. as shown in Figure 3, more than one pressure gradient may be calculated, e.g. between the first sensing portion S₁ and the second sensing portion S₂, between the third sensing portion S₃ and the first sensing portion S₁, between the third sensing portion S₃ and the second sensing portion S₁, thereby allowing for a finer granularity and allowing for a more precise location information where a blockage may exist.

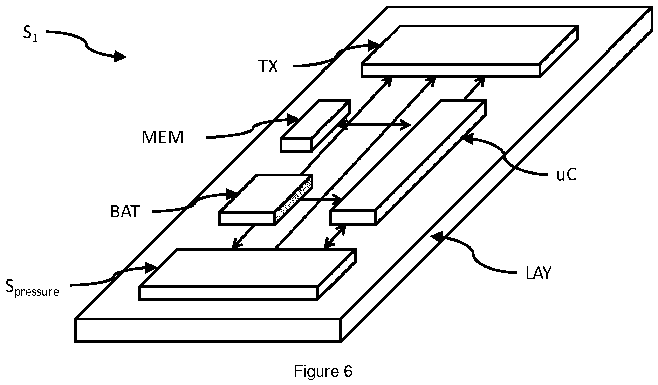

A sensor portion according to the invention is shown schematically in Figure 6.

The tissue stent 1 according to the invention may also comprises an energy harvesting and storage portion BAT, as well as a wireless sending portion TX for sending information indicative of the functional conditions of the passage, whereby the at least two sensing portions S₁, S₂ and the wireless sending portion TX are energized via the energy harvesting and storage section BAT.

The tissue stent 1 according to the invention also comprises an inductivity L₁The inductivity L₁ is temporarily connected to the energy harvesting and storage portion BAT allowing for energy harvesting. In times the inductivity L₁ is not connected to the energy harvesting and storage portion BAT the inductivity L₁ may be used for one or more other purposes. In particular, the inductivity L₁ may be temporarily connected to the wireless sending portion TX allowing for sending information. That is, the inductivity L₁ is emitting pulses according to a prescribed scheme representing information of the tissue stent 1.

Unlike conventional stent grafts, the tissue stent 1 according to the invention provides for an in-situ measurement which does not afford a highly trained medical practitioner.

Generally the tissue stent 1 according to the invention allows for measurement of the pressure at the inflow IN of the passage and the exit OUT. Based on both pressure data a pressure difference may be calculated (within the tissue stent or externally) thereby allowing to determine whether the tissue stent is still functional, partially blocked or fully blocked.

It is to be noted that the design of a tissue stent having a multi-purpose inductivity leads to a decreased size. This decreased size allows for easy transport towards a desired location of use as well as allows for enhanced measurement functionality.

As such the tissue stent according to the invention allows for simplified and reliable controls at lower costs.

The Tissue stent according to embodiments of the invention may be covered with a biocompatible respectively bioresistive cover COV, in particular a PTFE-cover. Biocompatible may also encompass that the cover COV exhibits certain additional properties as required, e.g. inhibiting ingrowth, releasing drugs, etc.

This cover COV may be arranged such that a sensing portion S₁, S₂,.. may even be partially covered by the cover COV and may spare the actual pressure sensing area Sₚᵣₑₛₛᵤᵣₑ as can be seen e.g. in figures 1-3 and 5.

As shown in figures 1 to 3 a sensing portion S₁ and S₂ is arranged near/at opposite ends of the stent 1. The sensing areas may be arranged at bare ends such that the pressure sensor Sₚᵣₑₛₛᵤᵣₑ is in contact with the fluid, e.g. arranged such that a fluid within the passage may pressure the pressure sensor through a mesh structure forming a supporting body MESH of the tissue stent 1. I.e. the tissue stent 1 is not completely covered by the cover COV but only partially, in particular in the middle section. Such a cover COV thereby allows covering and sealing electronic components of the sensing portions other than the actual pressure sensor while still allowing for deployment of a stent and providing superior flexibility. E.g. the supporting body MESH may be formed of a Nitinol like material having a certain number of cross struts.

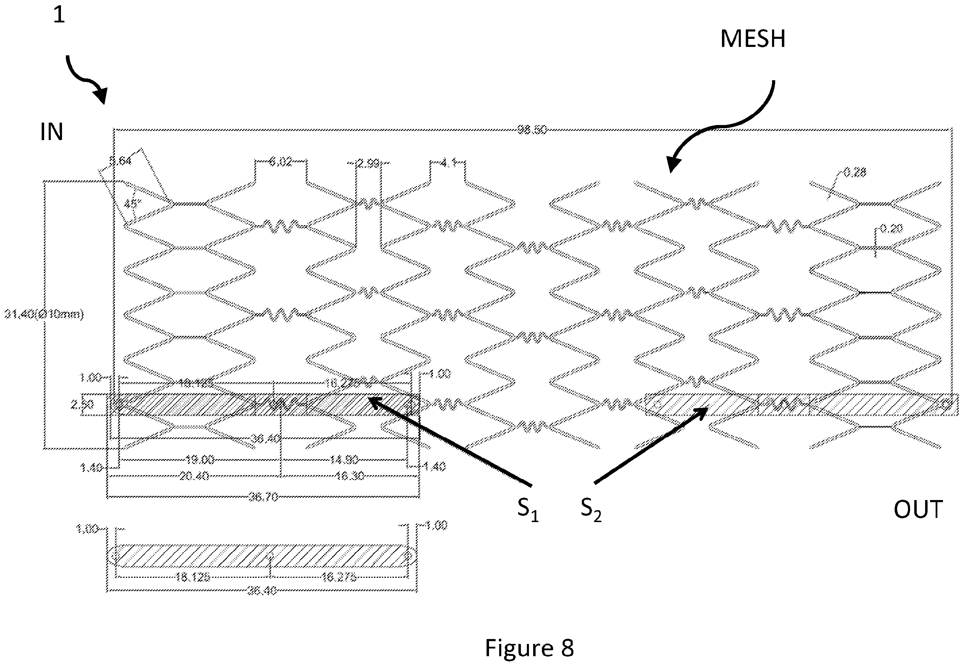

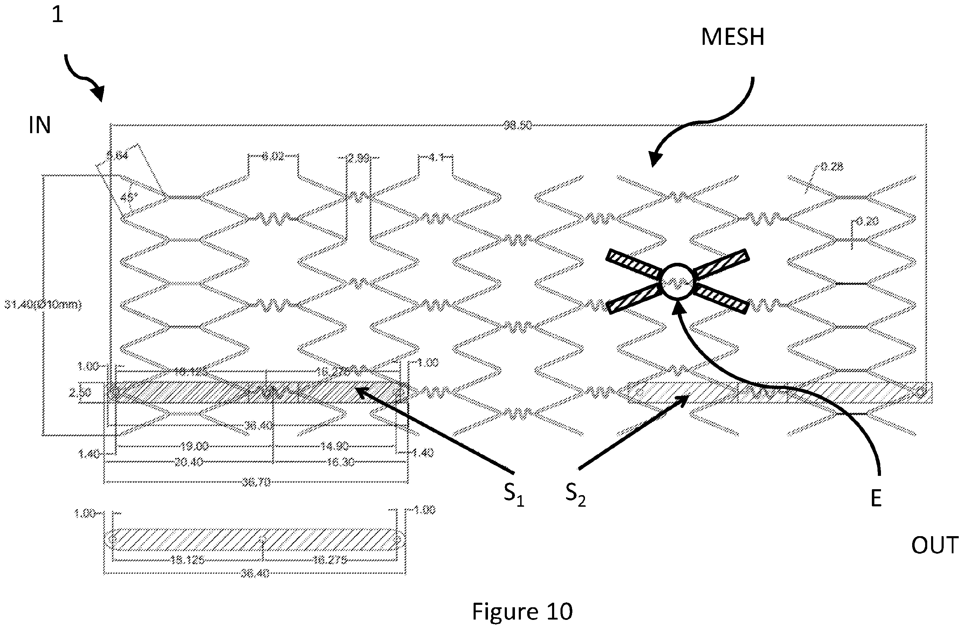

A tissue stent according to the invention may comprise e.g. 33 cross struts and 10 crowns like the one shown in Figure 8 and 10. The struts in between the crowns are designed such that they are short and flexible in the center region of the tissue stent 1 while being rigid towards the ends IN, OUT of the tissue stent 1. Thereby, the tissue stent 1 may be bend in the center region allowing for flexibility within the tissue while the end portions IN, OUT provide for stability e.g. for placement of the sensing portions S₁, S₂.

According to embodiment of the invention the wireless sending portion TX is arranged to harvest energy provided by external electro-magnetical fields.

I.e. the wireless sending portion may use an antenna / wire loop to collect electro-magnetical fields. This antenna / wire loop may be the same as used for sending information but it may also be a different antenna / wire loop for this particular purpose only.

In a further embodiment of the invention, the invention provides for an evaluation unit uC, such as a microcontroller, a microprocessor, an application-specific integrated circuit (ASIC) or a Field Programmable Gate Array (FPGA). Such an evaluation unit uC allows for evaluating sensor data received from the respective sensing portions before sending information. E.g. the evaluation unit may derive information indicative of the functional conditions of the passage, e.g. a pressure difference / a pressure gradient, before transmitting the information, i.e. the pressure difference / pressure gradient, via the wireless sending portion TX.

In an embodiment the wireless sending portion TX and the evaluation unit uC may be embodied in a single unit. This combined unit will also be identified as sending portion TX.

As such the tissue stent 1 may be arranged such that it may easily deployed via microsurgical devices and or intra-luminal deployment technologies such as stent advancement via a catheter and expansion of the stent 1 at the required location, i.e. the stent 1 is arranged for being expanded during the implantation process. Therefore, the stent 1 may comprise a mesh like supporting body MESH.

To allow for a simplified and cost-effective production, the sensing portions TX and / or the wireless sending portion TX as well as other portions of the sensor portion may be arranged on a flexible supporting layer LAY. This supporting layer is preferably flexible and may be a flexible printed circuit board. This supporting layer LAY together with all pre-assembled portions may then be arranged at specific positions relative to the supporting body MESH before a cover COV is applied which thereby allows for fixating the supporting layer and at least some of the portions arranged on the supporting layer LAY. Hence, the production may be streamlined in that pre-assembled sensing portions S₁, S₂, ... may be pre-produced on respective supporting layers LAY. The supporting layer LAY may allow for some bending of the tissue stent when being delivered such that tissue stent deployment is not detrimentally affected by the provisioning of the sensing portions. E.g. the supporting layer LAY may comprise a thin flexible polymeric sheet, e.g. made of / comprising polyimid. The supporting layer LAY may also be biocompatible respectively bioresistive.

According to embodiments of the invention the wireless sending portion TX is arranged for sending a unique identification ID. This ID may be unique with respect to a sensing portion or may be unique with respect to a tissue stent. Whether an ID is provided per stent or per sensing portion is subject to implementation. The purpose of the ID is to clearly identify each tissue stent. Hence, even if a patient is implanted with more than one tissue stent according to the invention, one would still be able to clearly recognize which information is originating from a particular stent.

In addition, some embodiments may comprise a storage portion MEM for storing information indicative of the functional conditions of the passage. Again, the information stored may be raw-data of the sensing portions, evaluated data such as obtained by an evaluation unit uC and the information may be stored such that the sensing portion of a particular data is derivable.

The stored information may be used for additional evaluations either by the evaluation unit uC or by an external unit after the data is sent thereto.

Consequently, the tissue stent may also allow for some interaction such that particular data may be forwarded.

The wireless sending portion TX may be any kind of near field communication technology. In particular it may conform to a Near Field Communication Standard. That is the system may be energized and read out in a distance of up 50 cm. Hence, a patient, respectively the tissue stents within the patient, may be read out contactless. Reading-out is thereby to be understood as data indicative to the actual status, i.e. raw-data and/or evaluated data, but may also relate to stored data and / or one or more unique ID.

The tissue stent 1 according to the invention is therefore particular suitable for the scenario mentioned in the background section, i.e. it may be used within a liver.

In essence, the tissue stent 1 according to the invention is not a prosthesis for a lumen (intra-luminal prosthesis) but is a stent for tissue. I.e. the channel the stent keeps open is created through a (micro-) surgical invention with the respective tissue. As such the tissue stent allows for a wireless inspection of the passage by providing data indicative of the passage.

In case of using the tissue stent 1 in a liver, the tissue stent may allow monitoring of pre- and post-hepatic pressure as well as flow properties and therefore allows objectively to control the passage kept open by the tissue stent.

Since the tissue stent 1 is equipped with an energy harvesting in storage portion BAT, the tissue stent requires no cables. Cables as known from other devices tend to create bridges allowing bacteria and virus entry. Hence eliminating such bridges allows for less complication.

Sensing portions may be fixed to the supporting body MESH e.g. via electro-spinning process as will be detailed in the following. A supporting layer LAY is arranged in between layers of cover COV. I.e. a first layer of cover material is electro-spun onto the body MESH, then the supporting layer as detailed above is applied onto this layer and another layer of cover material is electro-spun onto the covered body MESH and the supporting layer LAY thereby providing for a stable (adhesive) arrangement. Such a tissue stent 1 according to the invention may even be crimped towards a delivery system (geode wire, etc.) without harming the sensor portions.

As can be seen in figure 4, the sensing portions S₁, S₂ may be arranged on the inside (not shown) or the outside of the supporting body MESH.

The only requirement is that the pressure sensor Sₚᵣₑₛₛᵤᵣₑ of the sensing portion S₁, S₂, ... are able to detect a respective pressure at the location within the passage. Hence, it is not necessary that the pressure sensor is in fluidic communication with the passage. I.e. even the pressure sensor may be covered if the cover COV does allow for sensing pressure below the cover COV. Therefore, the production is inexpensive as stripping or shielding of the cover COV at the pressure sensor Sₚᵣₑₛₛᵤᵣₑ is obviated.

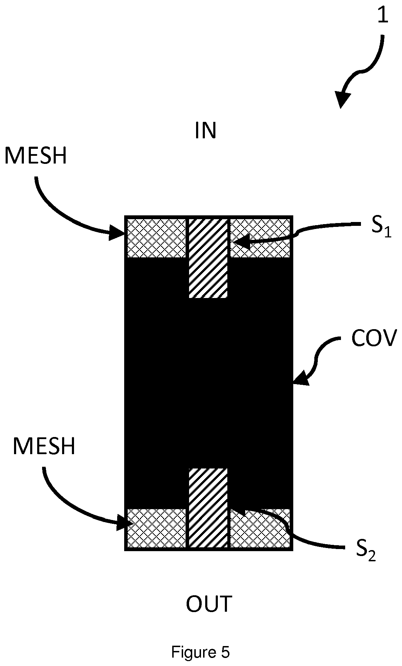

The supporting layer LAY as can be seen in figures 5, 6, 8 and 10 is preferably an elongated small strip which is provided preferably in parallel to an axis of the passage on top of the supporting body MESH. The supporting structure may provide for added flexibility by providing portions of even more reduced size such that more bending is allowed in certain regions thereby adding for the flexibility of the overall tissue stent 1.

As can be seen in Figure 8 and 10 in the bottom portion, the supporting layer LAY of a sensor portion may comprise one or more securing means such as holes allowing for securing the supporting layer LAY towards the supporting body either in addition to or as an alternative to an adhesive layer of the cover COV ontop of the supporting body MESH as described before.

I.e. the securing means allows for suturing the supporting layer towards the supporting body MESH.

In addition, the supporting body MESH may provide for securing means allowing for securing the end portions of the tissue sent 1 towards vessels and/or tissue. For example the supporting body MESH may provide for holes allowing for suturing the tissue stent 1 towards vessels and/or tissue.

The supporting layer LAY is preferably arranged substantially parallel with respect to an axis formed by the passage of the tissue stent 1. Thereby (as shown in Figures 8 and 10) the supporting layer LAY does not inhibit compression / decompression of the tissue stent 1 for delivery. To allow for such an arrangement, the supporting layer LAY may be fixed to the mesh MESH at one or more locations, whereby fixing may also encompass flexible material to accommodate displacement of fixing points of the mesh when being compressed / decompressed.

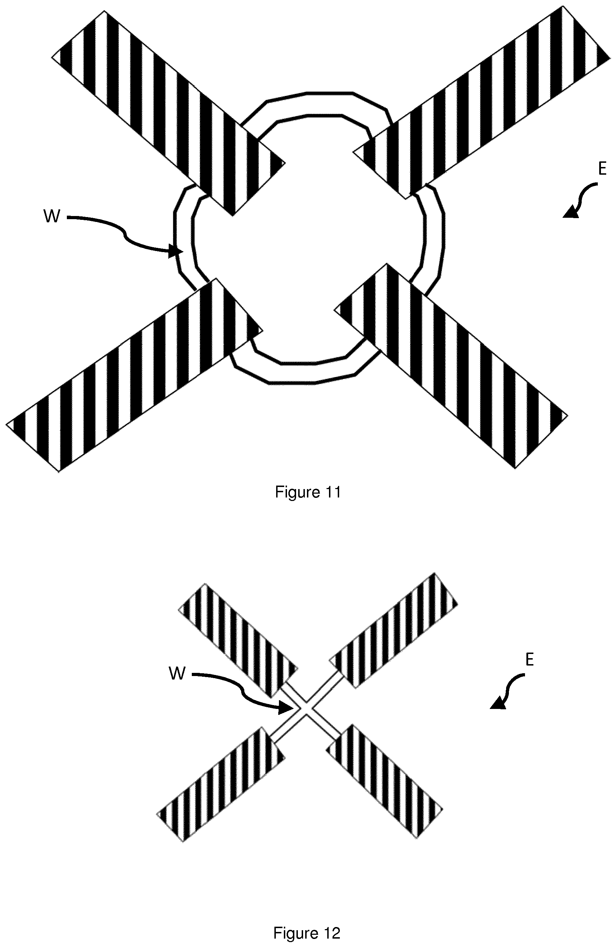

Furthermore, the supporting layer LAY may provide for a different shape as will be also further detailed with respect to Figures 10 and 11.

Preferably the components of the sensing portion are miniaturized. In particular surface mounted devices (SMD) may be used. Also some of the functionality, e.g. the evaluation unit and / or the memory and / or the transmission unit and / or the energy harvesting unit may be integrated into a chip or a chip arrangement or in a system in package (SIP) arrangement.

In particular, SMD inductivities may be used for the energy harvesting and storage portion BAT. Also bare dies, i.e. electronic elements without their typical housing, may be used allowing for a reduced height and increased flexibility. This is possible as the covering COV is protecting the dies from any harmful impact. Alternatively or in addition the electronics of the sensing portions may also be covered by another polymer or a silicone type covering.

To allow for increased range of the transmission of data / information, the sensing portions may allow for relative high voltages, e.g. provided by a charge pump and/or by means of inductivities.

Energy harvesting may be performed e.g. by arranging one or more inductivities L1, L2. Preferably a plurality of inductivities is provided.

It is furthermore preferred when providing multiple inductivities for energy harvesting that the directions of main sensitivity of the multiple inductivities are arranged such that the directions are not in parallel (but preferably orthogonal to each other) to ensure that irrespective of the main direction of energy provided by a "receiving unit" (a maximum of) energy may be harvested.

Furthermore, the inductivities may be selected such that they are part of a resonant circuit having a like resonant frequency as the frequency provided by the "receiving unit". The resonant frequency may also be used to generate a clock signal for the electronic E / sensors using inverters.

The harvested energy may be stored in one or more high-C capacitors (arranged in parallel).

To ensure smooth operation of the circuitry provided on the tissue stent further up- and/or down conversion of the voltage stored in said high-C capacitors may be provided to offer one or more constant voltage towards electronic components of the tissue stent necessitating such constant voltage.

High voltages may also be beneficially used for communication by the tissue stent 1. E.g. the data communication may be provided by high voltage pulses having a small pulse width of e.g. 1 µs. The duration is selected such that inductivity L₁, L₂ is charged (near to) maximum energy. The falling edge is such that for a sufficient long time a high voltage is provided at the inductivity. E.g. the high voltage may be around and even above 100 V. After decline of the voltage the next pulse is transmitted. To enable such high voltages the electronics E or at least parts thereof may be produced in a Highvoltage-CMOS process.

The system design allows for a coding of the data / information for added reliability of the transmission. Such coding may encompass error detection such as cyclic redundancy checks and error correction such as forward error correction schemes. However the use of higher coding schemes such as turbo coding is thereby not excluded. Preferably such issues are subject to higher layers of a respective data transmission protocol.

The system may even allow for training sequences and a coding negotiation of the wireless sending portion TX towards a data receiving unit. The receiving unit may even be arranged to instigate retransmission of data / information upon detecting errors and may even instruct a different, e.g. a more reliable, transmission scheme. There might be a plurality of transmission schemes having different properties which are subject to negotiation of the communicating entities. Preferably such issues are subject to higher layers of a respective data transmission protocol.

E.g. to enable transfer at large distances one may foresee that in a first data transmission data is send with error correction and even error detection bits added such that minor errors may still be detected. When the receiving portion detects errors which may not resolved the receiving portion may instruct the tissue stent 1 to retransmit some or all data with a higher error detection property. Obviously, subject to the error detection scheme this may adaptively escalated or de-escalated as needed to accommodate a respective channel.

Alternatively or in addition the impedance of the wireless energy transmission towards the energy harvesting BAT may be such that a control circuit controls MOS-varactors that a maximum voltage in the energy harvesting section (resonant circuit) is achieved. In an embodiment, it may be provisioned that in case the voltage exceeds a certain threshold, the tissue stent 1 may signal this situation (or may even indicate a norm indicative of the excess) such that the "data receiving unit" may reduce its transmission power.

As the electronics E is low-power and due to a packetized data communication having small clock cycles, the harvested energy may allow for autonomous operation of the tissues stent for long periods. That ist, even though the patient is out of reach of a receiving unit, the harvested energy allows for autonomous operation of the stent for time periods in the range of minutes, to hours and even several days.

It is noted that data transmission from the receiving unit towards the tissue stent may by repeated within a multiple period of the resonant period of the receiving resonant circuit. In doing so, energy harvesting is enabled while ensuring reception of the data.

Data measured by sensors may be stored in a non-volatile memory. Storage may be bitwise. To minimize storage it is preferred to store deviations of following values with respect to an initial stored value / threshold instead of absolute values to better utilize storage resources.

As the tissue stent 1 may offer a plurality of sensors it is preferred that the signals may be multiplexed. That is the sensors may be powered and measured individually. The measurements may be performed in a periodic manner or upon certain events. A particular sequence of measurements may also be foreseen. That is, the retrieval of sensor data is extremely flexible. In a flexible arrangement care must be taken to store sensor data appropriately (as well as to communicate sensor data appropriately) such that the sensor data may still be attributed towards a specific sensor. This also allows for reduced energy consumption.

To allow for multiple tissue stents 1 to communicate with a receiving unit (e.g. stents of the same patient and/or stents of a plurality of patients) the sending period of the tissue stents 1 may be triggered by a targeted request to a particular tissue stent or by a different time basis provided for each tissue stent. E.g. the time basis is determined by the start of operation of a respective tissue stent whereby the time frame used for communication is rather small compared to the interval between consecutive time frames. That is the likelihood of a collision of data is minimized.

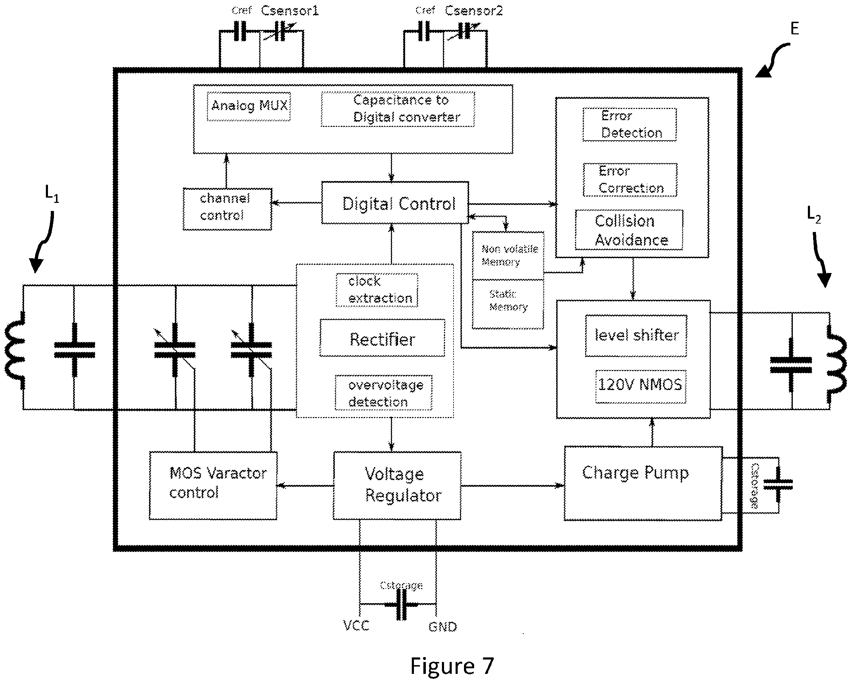

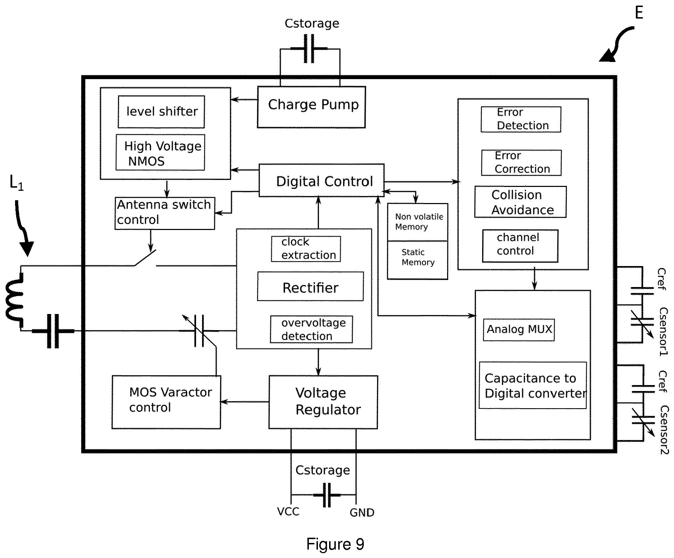

The energy harvesting and storage portion BAT may allow for inductive coupling. Therefore, the an energy harvesting and storage portion BAT may comprise as shown in Figure 7 or 9 a plurality of inductivities L₁, L₂ arranged within resonant circuits. It may even be foreseen that energy transmission towards the energy harvesting and storage portion BAT is performed will transmitting data from the "receiving unit" outside the patient towards the sensing portion(s) of a tissue stent 1 inside the patient.

The circuitry of an embodiment of the sensing portions may even allow for adaption of the transmitted power, e.g. by measuring a voltage attained within the sensing portion by means of the energy harvesting and storage portion BAT and if the voltage attains or exceeds a certain threshold, it is indicated towards the "receiving unit" that the power transmitted towards the an energy harvesting and storage portion BAT may be reduced.

The energy harvesting and storage portion BAT as shown in figures 7 and 9 may comprise one or more capacities, in particular having high capacity. The storage capacity may be increased by usage of capacities in parallel.

The energy provided by the energy harvesting and storage portion BAT is sufficient that the sensing portion may operate autonomously for a certain period of time allowing for repowering the energy harvesting and storage portion BAT in intervals, thereby allowing a patient to perform a normal life through the majority of time. To accommodate different charging situations, the sensing portion may comprise a unit for up and/or down conversion such that the required voltage by the other units may be provided as necessary in a flexible manner.

In the present invention shown in Figure 9 in contrast to the embodiment shown in figure 7 only needs a single coil / inductivity L₁ for plural functions. In this embodiment, the same coil / inductivity L₁ may be used for data transmission as well as for energy harvesting. To enable such operation a block "antenna switch control" is provided. It is noted that in most cases it is not necessary to harvest energy and to communicate at the same time. By a respective type of communication towards the stent also energy may be transferred along with the communication. Nevertheless whether the coil / inductivity L₁ is used in parallel or in an alternating manner for said purposes it is of particular relevance that the size of the stent may be reduced dramatically, e.g. by about 1/3 compared to a double coil / inductivity arrangement of Figure 7, where each coil / inductivity L₁ andL₂ has a specific purpose. When using a coil / inductivity L₁ of the energy harvesting BAT as a sending coil / inductivity it is however necessary to provide an energy storage which may be embodied by a capacitor C_{storage} as indicated in Figure 9.

The memory MEM shown in Figure 6 may be of a volatile or a non-volatile type. In particular the memory MEM may comprise an EE(P)ROM or Flash memory. Storing of data allows for a packetized transfer of a plurality of data towards a receiving unit thereby increasing energy efficiency.

The storage of data as well as the transfer of data may be adapted to gain further efficiency e.g. by storing only deviation with respect to a preceding value or with respect to an initial value.

In Figure 10 and 11 and 12 another aspect of the invention is shown. In order to enable easy placement in a human body, the tissue stent may be shrinked, e.g. by crimping, for delivery.

As some of the electronics E (e.g. as shown in figure 7 and 9) of the tissue stent 1, i.e. parts of said at least two sensing portions S₁, S₂, said energy harvesting and storage portion BAT, and said wireless sending portion TX, are rather brittle and/or inflexible, it is preferred to arrange / fix such parts substantially in parallel to a portion of the mesh MESH such that they may be moved substantially with the mesh portion during expansion / compression.

Thereby it is enabled to shrink the electronic E for delivery and to expand the electronic once in place.

Mesh portions used for arrangement / fixing of portions of the electronics E are preferably inflexible compared to portions of the mesh acting as knots (crowns) of the mesh MESH.

It is noted that said parts are fixedly connected to said respective portion of the mesh MESH each at one particular location.

Although one fixation of each art towards a respective portion may by sufficient, it may also be provided that more than one fixation location is present. In that case - to allow shrinking / expanding of the respective portion of the mesh MESH, the additional location allows for movement in parallel to the respective portion of the mesh MESH during expansion / compression. To enable such operation the fixation may be movable with respect to the mesh portion and/or the fixation material may offer flexibility.

Obviously, in such a portioned arrangement of the electronics E the respective electrical connections across portions are preferably flexible, e.g. wires W, so that they may bend when the tissue stent is compressed / expanded / delivered.

As stated before, the invention may use any kind of near field communication system. I.e. the invention may even allow a patient to read out / energize his or her stent via a near field communication enabled home device, e.g. a mobile phone having a NFC interface, as an exemplary receiving unit. The data received indicative of the passage may then be processed and/or stored and/or forwarded towards a medical center for further analysis respectively generation of warning messages in case of a suspected blockage of the passage which might necessitate a re-intervention.

## Claims

1. Tissue stent (1) for allowing passage of a fluid, the tissue stent (1) being suited for implantation into the body of a mammal, the tissue stent (1) comprising:
• a body (MESH) forming said passage from an entry side (IN) towards an exit side (OUT) when implanted,
• an energy harvesting and storage portion (BAT),
• an inductivity (L₁),
**characterized in that** the tissues stent also comprises
• at least two sensing portions (S₁, S₂), whereby a first sensing portion (S₁) is arranged near the entry side (IN) and whereby a second sensing portion (S₂) is arranged near the exit side (OUT), the first sensing portion (S₁) and second sensing portion (S₂) being pressure sensors (Sₚᵣₑₛₛᵤᵣₑ),
• a wireless sending portion (TX) for sending information indicative of the functional conditions of the passage,
• whereby the at least two sensing portions (S₁, S₂) and the wireless sending portion (TX) are energized via the energy harvesting and storage section (BAT),
• whereby the inductivity (L₁) is temporarily connected to the energy harvesting and storage portion (BAT) allowing for energy harvesting, and whereby the inductivity (L₁) when not connected to the energy harvesting and storage portion (BAT) is temporarily connected to the wireless sending portion (TX) allowing for sending information.

2. Tissue stent according to claim 1, whereby the tissue stent is covered with a biocompatible cover (COV), in particular a PTFE-cover.

3. Tissue stent according to claim 2, whereby parts of the sensing portions (S₁, S₂) are arranged within the biocompatible cover (COV).

4. Tissue stent according to anyone of claims 1 to 3, whereby the wireless sending portion (TX) is arranged to harvest energy provided by external electro-magnetical fields.

5. Tissue stent according to anyone of claims 1 to 4, whereby the wireless sending portion (TX) is further arranged to evaluate sensor data received from the first and second sensing portion (S₁,S₂) before sending information indicative of the functional conditions of the passage.

6. Tissue stent according to anyone of claims 1 to 5, whereby the tissue stent (1) is arranged for being expanded during the implantation process.

7. Tissue stent according to anyone of claims 1 to 6, whereby the sensing portions (TX) and / or the wireless sending portion (TX) are arranged on a flexible supporting layer (LAY).

8. Tissue stent according to anyone of claims 1 to 7, whereby the wireless sending portion (TX) is arranged for sending a unique identification (ID).

9. Tissue stent according to anyone of claims 1 to 8, further comprising a storage portion (MEM) for storing information indicative of the functional conditions of the passage.

10. Tissue stent according to anyone of claims 1 to 9, whereby the wireless sending portion (TX) is conforming to a Near Field Communication Standard.

11. Tissue stent according to anyone of claims 1 to 10, whereby at least parts of said at least two sensing portions (S₁, S₂), said energy harvesting and storage portion (BAT), and said wireless sending portion (TX) are arranged on different portions, whereby each portion is arranged substantially in parallel to a portion of the mesh (MESH) such that they may be moved substantially with the respective mesh portion during expansion / compression.

12. Tissue stent according to claim 11, whereby said parts are interconnected by flexible wires.

13. Tissue stent according to claim 11 or 12, whereby said parts are fixedly connected to said respective portion of the mesh (MESH) each at one particular location.

14. Tissue stent according to claim 13, whereby said parts are connected to said respective portion of the mesh (MESH) each at another particular location, whereby the connection allows for movement in parallel to the respective portion of the mesh (MESH) during expansion / compression.

15. Tissue stent according to anyone of claims 1 to 14, whereby in case of a failure in communication the wireless sending portion (TX) is adapted to adapt communication using a different coding scheme allowing for error correction.

16. Tissue stent according to anyone of claims 1 to 15, whereby said energy harvesting and storage portion (BAT) comprises one or more capacitors having high capacity.

17. Tissues stent according to anyone of claims 1 to 16, whereby a third sensor S₃ is provided in between the first sensing portion S₁ and the second sensing portion S₂, thereby allowing to calculate more than one pressure gradient, thereby allowing for a finer granularity and allowing for a more precise location information where a blockage may exist.

18. Tissues stent according to anyone of claims 1 to 17, whereby the supporting body (MESH) provides securing means allowing for securing an end portion of the tissue stent (1) towards vessels and/or tissue.

19. Tissues stent according to anyone of claims 1 to 18, whereby another inductivity (L₂) is provided, whereby the directions of main sensitivity of the inductivities (L₁, L₂) are arranged such that the directions are not in parallel.

20. Tissues stent according to anyone of claims 1 to 19, whereby the tissue stent further comprises a control circuit and MOS-varactors, whereby the control circuit is arranged to control the MOS-varactors such that a maximum voltage in the energy harvesting and storage portion (BAT) is achieved, whereby in case the voltage exceeds a certain threshold, the tissue stent 1 may signal this situation such that an external unit receiving information indicative of the functional conditions of the passage may reduce its transmission power.

21. Tissues stent according to anyone of claims 1 to 20, whereby the signals of the at least two sensing portions (S1, S2), are multiplexed.

22. Tissues stent according to anyone of claims 1 to 21, whereby the first sensing portion comprises a unit for up and/or down conversion such that the required voltage by the other units may be provided as necessary in a flexible manner.

## Patentansprüche

1. Gewebestent (1) zur Ermöglichung des Durchgangs eines Fluids, wobei der Gewebestent (1) zur Implantation in den Körper eines Säugetiers geeignet ist, wobei der Gewebestent (1) umfasst:
• einen Körper (MESH), der den Durchgang von einer Eingangsseite (IN) zu einer Ausgangsseite (OUT) bildet, wenn er implantiert ist,
• einen Abschnitt zur Energiegewinnungs und -speicherung (BAT),
• eine Induktivität (L₁),
**dadurch gekennzeichnet, dass** der Gewebestent ferner umfasst
• mindestens zwei Sensorabschnitte (S₁, S₂), wobei ein erster Sensorabschnitt (S₁) in der Nähe der Eingangsseite (IN) und ein zweiter Sensorabschnitt (S₂) in der Nähe der Ausgangsseite (OUT) angeordnet ist, wobei der erste Sensorabschnitt (S₁) und der zweite Sensorabschnitt (S₂) Drucksensoren (S_{Druck}) sind,
• einen drahtlosen Sendeabschnitt (TX) zum Senden von Informationen, die den Funktionszustand des Durchgangs anzeigt,
• wobei die mindestens zwei Sensorabschnitte (S₁, S₂) und der drahtlose Sendeabschnitt (TX) über den Energiegewinnungs- und -speicherabschnitt (BAT) mit Energie versorgt werden,
• wobei die Induktivität (L₁) vorübergehend mit dem Energiegewinnungs- und - speicherabschnitt (BAT) verbunden ist, was eine Energiegewinnung ermöglicht, und wobei die Induktivität (L₁), wenn sie nicht mit dem Energiegewinnungs- und -speicherabschnitt (BAT) verbunden ist, temporär mit dem drahtlosen Sendeabschnitt (TX) verbunden ist, um das Senden von Informationen zu ermöglichen.

2. Gewebestent nach Anspruch 1, wobei der Gewebestent mit einer biokompatiblen Abdeckung (COV), insbesondere einer PTFE-Abdeckung, bedeckt ist.

3. Gewebestent nach Anspruch 2, wobei Abschnitte der Sensorabschnitte (S₁, S₂) innerhalb der biokompatiblen Abdeckung (COV) angeordnet sind.

4. Gewebestent nach einem der Ansprüche 1 bis 3, wobei der drahtlos Sendeabschnitt (TX) so angeordnet ist, dass er von externen elektromagnetischen Feldern bereitgestellte Energie gewinnt.

5. Gewebestent nach einem der Ansprüche 1 bis 4, wobei der drahtlose Sendeabschnitt (TX) ferner so angeordnet ist, dass er Sensordaten auswertet, die von dem ersten und zweiten Sensorabschnitt (S₁, S₂) empfangen werden, bevor er Informationen sendet, die den Funktionszustand des Durchgangs anzeigen.

6. Gewebestent nach einem der Ansprüche 1 bis 5, wobei der Gewebestent (1) so angeordnet ist, dass er während des Implantationsprozesses ausgedehnt wird.

7. Gewebestent nach einem der Ansprüche 1 bis 6, wobei die Sensorabschnitte (TX) und/oder der drahtlose Sendeabschnitt (TX) auf einer flexiblen Trägerschicht (LAY) angeordnet sind.

8. Gewebestent nach einem der Ansprüche 1 bis 7, wobei der drahtlose Sendeabschnitt (TX) zum Senden einer eindeutigen Kennung (ID) angeordnet ist.

9. Gewebestent nach einem der Ansprüche 1 bis 8, ferner umfassend einen Speicherabschnitt (MEM) zum Speichern von Informationen, die den Funktionszustand des Durchgangs anzeigen.

10. Gewebestent nach einem der Ansprüche 1 bis 9, wobei der drahtlose Sendeabschnitt (TX) einem Nahfeldkommunikationsstandard entspricht.

11. Gewebestent nach einem der Ansprüche 1 bis 10, wobei zumindest Abschnitte der mindestens zwei Sensorabschnitte (S₁, S₂), des Energiegewinnungs- und - speicherabschnitts (BAT) und des drahtlosen Sendeabschnitts (TX) auf verschiedenen Abschnitten angeordnet sind, wobei jeder Abschnitt im Wesentlichen parallel zu einem Abschnitt des Netzes (MESH) angeordnet ist, so dass sie während der Ausdehnung/Kompression im Wesentlichen mit dem jeweiligen Netzabschnitt bewegt werden können.

12. Gewebestent nach Anspruch 11, wobei die Abschnitte durch flexible Drähte miteinander verbunden sind.

13. Gewebestent nach Anspruch 11 oder 12, wobei die Abschnitte mit dem jeweiligen Abschnitt des Netzes (MESH) jeweils an einer bestimmten Stelle fest verbunden sind.

14. Gewebestent nach Anspruch 13, wobei die Abschnitte mit dem jeweiligen Abschnitt des Gewebes (MESH) jeweils an einer anderen bestimmten Stelle verbunden sind, wobei die Verbindung eine Bewegung parallel zu dem jeweiligen Abschnitt des Gewebes (MESH) während der Ausdehnung/Kompression ermöglicht.

15. Gewebestent nach einem der Ansprüche 1 bis 14, wobei im Falle einer Störung der Kommunikation der drahtlose Sendeabschnitt (TX) so angepasst ist, dass er die Kommunikation unter Verwendung eines anderen Codierungsschemas, das eine Fehlerkorrektur ermöglicht, anpassen kann.

16. Gewebestent nach einem der Ansprüche 1 bis 15, wobei der Energiegewinnungs- und - speicherabschnitt (BAT) einen oder mehrere Kondensatoren mit hoher Kapazität umfasst.

17. Gewebestent nach einem der Ansprüche 1 bis 16, wobei ein dritter Sensor S₃ zwischen dem ersten Sensorabschnitt S₁ und dem zweiten Sensorabschnitt S₂ vorgesehen ist, wodurch mehr als ein Druckgradient berechnet werden kann, wodurch eine feinere Granularität und eine genauere Ortsangabe ermöglicht wird, wo eine Blockade vorhanden sein kann.

18. Gewebestent nach einem der Ansprüche 1 bis 17, wobei der Stützkörper (MESH) Befestigungsmittel aufweist, die es ermöglichen, dass ein Endabschnitt des Gewebestents (1) an Gefäßen und/oder Gewebe befestigt wird.

19. Gewebestent nach einem der Ansprüche 1 bis 18, wobei eine weitere Induktivität (L₂) vorgesehen ist, wobei die Richtungen der Hauptempfindlichkeit der Induktivitäten (L₁, L₂) so angeordnet sind, dass die Richtungen nicht parallel sind.

20. Gewebestent nach einem der Ansprüche 1 bis 19, wobei der Gewebestent ferner einen Steuerschaltkreis und MOS-Varaktoren umfasst, wobei der Steuerschaltkreis so angeordnet ist, dass er die MOS-Varaktoren so steuert, dass eine maximale Spannung in dem Energiegewinnungs- und -speicherabschnitt (BAT) erreicht wird, wobei, falls die Spannung einen bestimmten Schwellenwert überschreitet, der Gewebestent 1 diese Situation signalisieren kann, so dass eine externe Einheit, die eine externe Einheit, die Informationen über den Funktionszustand des Durchgangs empfängt, ihre Sendeleistung reduzieren kann.

21. Gewebestent nach einem der Ansprüche 1 bis 20, wobei die Signale der mindestens zwei Sensorabschnitte (S₁, S₂) multiplexiert werden.

22. Gewebestent nach einem der Ansprüche 1 bis 21, wobei der erste Sensorabschnitt eine Einheit zur Aufwärts- und/oder Abwärtswandlung umfasst, so dass die erforderliche Spannung von den anderen Einheiten je nach Bedarf auf flexible Weise bereitgestellt werden kann.

## Revendications

1. Stent tissulaire (1) permettant le passage d'un fluide, le stent tissulaire (1) étant adapté pour une implantation dans le corps d'un mammifère, le stent tissulaire (1) comprenant :
• un corps (MESH) formant ledit passage d'un côté d'entrée (IN) vers un côté de sortie (OUT) une fois implanté,
• un élément de récupération et stockage d'énergie (BAT),
• un inducteur (L₁),
**caractérisé en ce que** le stent tissulaire comprend également
• au moins deux éléments de détection (S₁, S₂), un premier élément de détection (S₁) étant disposé près du côté entrée (IN) et un deuxième élément de détection (S₂) étant disposé près du côté sortie (OUT), le premier élément de détection (S₁) et le deuxième élément de détection (S₂) étant des capteurs de pression (Sₚᵣₑₛₛᵤᵣₑ),
• un élément d'envoi sans fil (TX) pour envoyer des informations indicatives des conditions fonctionnelles du passage,
• les au moins deux éléments de détection (S₁, S₂) et l'élément d'envoi sans fil (TX) étant alimentés par l'élément de collecte et de stockage d'énergie (BAT),
• l'inducteur (L₁) étant temporairement connecté à l'élément de collecte et de stockage d'énergie (BAT) permettant la collecte d'énergie, et l'inducteur (L₁), lorsqu'il n'est pas connecté à l'élément de collecte et de stockage d'énergie (BAT), étant temporairement connecté à l'élément d'envoi sans fil (TX) permettant l'envoi d'informations.

2. Stent tissulaire selon la revendication 1, le stent tissulaire étant recouvert d'une enveloppe biocompatible (COV), en particulier d'une enveloppe en PTFE.

3. Stent tissulaire selon la revendication 2, des parties des éléments de détection (S₁, S₂) étant disposées à l'intérieur du couvercle biocompatible (COV).

4. Stent tissulaire selon l'une quelconque des revendications 1 à 3, dans lequel l'élément d'envoi sans fil (TX) est conçu pour collecter l'énergie fournie par des champs électromagnétiques externes.

5. Stent tissulaire selon l'une quelconque des revendications 1 à 4, dans lequel l'élément d'émission sans fil (TX) est en outre conçu pour évaluer des données de capteur reçues des premier et deuxième éléments de détection (S₁, S₂) avant d'envoyer des informations indicatives des conditions fonctionnelles du passage.

6. Stent tissulaire selon l'une quelconque des revendications 1 à 5, le stent tissulaire (1) étant conçu pour être dilaté pendant le processus d'implantation.

7. Stent tissulaire selon l'une quelconque des revendications 1 à 6, dans lequel les éléments de détection (TX) et/ou l'élément d'émission sans fil (TX) sont disposés sur une couche support flexible (LAY).

8. Stent tissulaire selon l'une quelconque des revendications 1 à 7, dans lequel l'élément d'envoi sans fil (TX) est conçu pour envoyer une identification unique (ID).

9. Stent tissulaire selon l'une quelconque des revendications 1 à 8, comprenant en outre un élément de stockage (MEM) pour stocker des informations indicatives des conditions fonctionnelles du passage.

10. Stent tissulaire selon l'une quelconque des revendications 1 à 9, dans lequel l'élément d'émission sans fil (TX) est conforme à une norme de communication en champ proche.

11. Stent tissulaire selon l'une quelconque des revendications 1 à 10, dans lequel au moins des parties desdits au moins deux éléments de détection (S₁, S₂), dudit élément de collecte et de stockage d'énergie (BAT) et dudit élément d'envoi sans fil (TX) sont disposées sur des parties différentes, chaque partie étant disposée sensiblement parallèlement à une partie de la maille (MESH) de sorte qu'elles peuvent être sensiblement déplacées avec la partie de la maille pendant l'expansion/la compression.

12. Stent tissulaire selon la revendication 11, dans lequel lesdites parties sont interconnectées par des fils flexibles.

13. Stent tissulaire selon la revendication 11 ou 12, dans lequel lesdites parties sont reliées de manière fixe à ladite partie correspondante de la maille (MESH), chacune à un emplacement particulier.

14. Stent tissulaire selon la revendication 13, dans lequel lesdites parties sont reliées à ladite partie correspondante de la maille (MESH), chacune à un autre emplacement particulier, la connexion permettant un mouvement parallèle à la partie respective de la maille (MESH) pendant l'expansion/la compression.

15. Stent tissulaire selon l'une quelconque des revendications 1 à 14, dans lequel, en cas de défaillance de communication, l'élément d'émission sans fil (TX) est apte à adapter la communication en utilisant un schéma de codage différent permettant la correction d'erreurs.

16. Stent tissulaire selon l'une quelconque des revendications 1 à 15, dans lequel ledit élément de collecte et de stockage d'énergie (BAT) comprend un ou plusieurs condensateurs ayant une capacité élevée.

17. Stent tissulaire selon l'une quelconque des revendications 1 à 16, dans lequel un troisième capteur S₃ est prévu entre le premier élément de détection S₁ et le deuxième élément de détection S₂, en permettant ainsi de calculer plus d'un gradient de pression, en permettant ainsi une granularité plus fine et en permettant une information de localisation plus précise là où un blocage peut exister.

18. Stent tissulaire selon l'une quelconque des revendications 1 à 17, dans lequel le corps support (MESH) crée des moyens de fixation permettant de fixer un élément d'extrémité du stent tissulaire (1) contre des vaisseaux et/ou des tissus.

19. Stent tissulaire selon l'une quelconque des revendications 1 à 18, dans lequel un autre inducteur (L₂) est prévu, les directions de sensibilité principale des inducteurs (L₁, L₂) étant disposés de telle sorte que les directions ne soient pas parallèles.

20. Stent tissulaire selon l'une quelconque des revendications 1 à 19, tandis que le stent tissulaire comprend en outre un circuit de commande et des varactors MOS, tandis que le circuit de commande est conçu pour commander les varactors MOS de telle sorte qu'une tension maximale dans l'élément de collecte et de stockage d'énergie (BAT) soit atteinte, tandis que, dans le cas où la tension dépasse un certain seuil, le stent tissulaire 1 peut signaler cette situation à une unité de réception externe, de sorte qu'un unité externe recevant des informations indiquant les conditions fonctionnelles du passage puisse réduire sa puissance de transmission.

21. Stent tissulaire selon l'une quelconque des revendications 1 à 20, dans lequel les signaux des au moins deux éléments de détection (S₁, S₂) sont multiplexés.

22. Stent tissulaire selon l'une quelconque des revendications 1 à 21, dans lequel le premier élément de détection comprend une unité pour une conversion ascendante et/ou descendante de telle sorte que la tension requise par les autres unités puisse être fournie de manière flexible si nécessaire.
